# EUROPEAN PATENT APPLICATION

(11) **EP 3 130 368 A1**
(43) Date of publication of application: **15.02.2017**
(21) Application number: 15179326.2
(22) Date of filing: 31.07.2015
(51) Int. Cl.: A61M 5/24, A61C 5/00, A61M 5/28, B65D 81/00, A61M 5/315, A61M 5/31

(54) **CHILD-RESISTANT DISCHARGER**

(71) Applicant: Sulzer Mixpac AG, 9469 Haag (Rheintal) (CH)
(72) Inventor: ZÜND, Marco, 9443 Widnau (CH)
(74) Representative: Manitz, Finsterwald & Partner GbR

(57) **Abstract**

The present disclosure relates to a discharger for discharging a predetermined amount of fluid and to a discharge system comprising the discharger. The discharger comprises a housing defining a longitudinal axis and having a proximal end and a distal end, a discharge section having a discharge passage for the fluid extending between an inlet opening and an outlet opening of the discharge section, a carriage cooperating with the proximal end region of the housing such that the carriage is moveable relative to the housing along the longitudinal axis, and securing means being engageable with each other such that they define a locking position in which the carriage is prevented from being moved along the longitudinal axis, the securing means being disengageable to define a starting position for the carriage by simultaneously applying a first actuating force to the housing and a second actuating force to the carriage.

## Description

The present disclosure relates to a child-resistant discharger for discharging a predetermined amount of fluid and to a discharge system comprising the discharger. The present disclosure also relates to the use of the discharger and the discharge system for discharging a fluid including at least one medical, dental or veterinary agent.

Generally, dischargers such as expendable syringes for use in the medical, dental or veterinary field are known which comprise one or more compartments in which the medical, dental or veterinary agent or a liquid including the medical, dental or veterinary agent is contained before use.

In many cases, such dischargers contain substances which can, if applied incorrectly, give rise to health hazards. In particular, ingestion or contact with the skin has to be prevented since otherwise poisoning or chemical burns of persons or animals can occur. Whereas adults are generally in control of the correct handling of such substances, particularly children are exposed to a high level of risk since they might consider the content of such applicators to be something edible and, in turn, ingest it.

In view of the foregoing it is an object of the present invention to provide a discharger for discharging a predetermined amount of fluid which, in particular, prevents children from unauthorized access to the fluid to be discharged. It is a further object to provide a discharger enabling an easy and reliable usage.

These objects are satisfied by a discharger having the features of claim 1 and a discharge system having the features of claim 14.

In accordance with the invention a discharger for discharging a predetermined amount of fluid comprises
- a housing defining a longitudinal axis and having a proximal end and a distal end,
- a discharge section having a discharge passage for the fluid extending between an inlet opening and an outlet opening of the discharge section,
- a carriage cooperating with the proximal end region of the housing such that the carriage is moveable relative to the housing along the longitudinal axis, and
- securing means being engageable with each other such that they define a locking position in which the carriage is prevented from being moved along the longitudinal axis, the securing means being disengageable to define a starting position for the carriage by simultaneously applying a first actuating force to the housing and a second actuating force to the carriage.

The carriage, in general, can take three main positions. In the "locking position" the carriage is at least not movable in an axial direction. Preferably, the carriage is fixed and immobile. The "starting position" defines the position of the carriage immediately after leaving the "locking position", i.e. after the simultaneous application of the two actuating forces leading to the disengagement of the securing means. In certain embodiments the "starting position" can be equated to a "discharge position", i.e. a position in which the liquid can readily be discharged, particularly by pushing the carriage into the housing. The "discharge position" particularly corresponds to an axial mobility of the carriage. However, in other embodiments, the carriage has to be moved further from the "starting position" into the "discharge position", preferably by further applying the second actuating force.

The child-resistant discharger, in general, may be adapted to be used in the medical, dental or veterinary field or in the health care sector. The fluid to be discharged is in particular a liquid which includes at least one medical, dental or veterinary agent. The present disclosure is, however, not limited to the medical, dental or veterinary sector and can be used in other appliances in which it is desirable to prevent an operator and in particular children from getting into contact with substances which in the event of incorrect use might represent a potential health threat.

The present disclosure is not limited to the discharge of fluids such as liquids. Rather, the child-resistant discharger as disclosed herein may alternatively be adapted or used to discharge viscous or gel-like materials.

The discharger in accordance with the invention requires the coordination of two different movement procedures, i.e. the application of two forces not pointing into the same direction. As a consequence, the discharger is resistant to discharge unless two different movement procedure and/or two different forces are applied to it. The risk of an inadvertent discharge due to an unintentional or accidental movement thereby is minimized or even prevented. Thus, the discharger in particular prevents an unintentional discharge of the fluid to be discharged. This is achieved by preventing the carriage from being unintentionally moved along the longitudinal axis in a manner allowing for a discharge of the liquid.

This locking mechanism that has to be released before the liquid can be discharged is under ordinary circumstances not surmountable by children, especially young children. As a consequence, the provided discharger can be regarded as being child-resistant or even essentially child-proof.

Preferably the first actuating force is brought about by a radial compression of the housing. In particular the radial compression is brought about at the housing's proximal region.

The radial compression of the housing preferably involves a squeezing operation, i.e. the housing becomes radially compressed on two essentially opposite sides. This could for example be achieved by holding the housing between thumb and index finger and pressing them together.

The second actuating force to the carriage is preferably brought about by a rotary motion or an axial motion of the carriage relative to the housing or a combination of both.

The direction of the rotary motion of the carriage can be either clockwise or counter clockwise, i.e. the carriage can be turned in either directions relative to the housing. Analogously, the axial motion can either point towards the distal end of the housing or away from it, i.e. the carriage can be pushed or pulled relative to the housing.

Expediently, the carriage is received in the proximal end region of the housing.

In a preferred design of the discharger the securing means are disengageable by radially compressing, in particular squeezing, the housing and simultaneously turning the carriage clockwise or counter clockwise. This mechanism can also be referred to as "squeeze-and-turn mechanism".

In an alternative design the securing means are disengageable by radially compressing, in particular squeezing, the housing and simultaneously pushing or pulling the carriage relative to the housing. This mechanism can also be referred to as "squeeze-and-push/pull mechanism".

In a particularly preferred embodiment, for disengaging purposes of the securing means, the housing has to be radially compressed, in particular squeezed, and the carriage has to be simultaneously rotated and pushed or pulled relative to the housing.

The securing means preferably comprise at least one locking element provided on the housing and at least one counter element provided on the carriage.

The locking element and the counter element preferably are protrusions extending from an inner and/or an outer side of the housing and the carriage, respectively. The protrusions may have a knob-like, pin-like, conus-like, sawtooth-like, ramp-like or polyhedral shape, for instance. Its cross-section may, for example, be essentially semicircular, triangular, rectangular, square or polygonal. The locking element and the counter element may be of different shape.

It is particularly preferred that the locking element is formed integrally with the housing and/or the counter element is formed integrally with the carriage.

Advantageously the locking element is adapted to slide over the counter element when the carriage is rotated relative to the housing in a first direction for arriving at the locking position, and wherein the locking element and the counter element prevent a rotation in the direction opposite to the first direction when they are engaged in the locking position.

In this connection it is preferred that either the locking element or the counter element is ramp-like in design such that sliding over of the corresponding other element in the first direction for arriving at the locking position requires rather low forces. The locking position is reached after the corresponding other element has passed the ramp, i.e. latched or snapped in. Due to the ramp-like design a movement in the opposite direction requires rather large forces or is even prevented and can only be achieved under normal conditions by a previous deformation of the housing.

In this connection it should be noted that the housing is preferably elastically deformable at least in those regions where the first actuating force is brought about. Expediently, the housing consists of elastically deformable polymers such as polypropylene. It is also conceivable that the carriage is at least partially elastically deformable and consists of elastically deformable polymers.

In an embodiment, the locking element or the counter element itself is elastically deformed when the corresponding other element is slid over in the first direction for arriving at the locking position. As an alternative or in addition, in another embodiment, an area of the housing or the carriage supporting the locking element or the counter element is elastically deformed.

In a particularly preferred embodiment, the housing of the discharger is deformable in order to disengage the locking element and the counter element by radially compressing the housing and rotating the carriage in the direction opposite to the first direction.

Advantageously, a pair of diametrically opposed locking elements is provided on the housing to engage/disengage with a pair of diametrically opposed counter elements provided on the carriage. To release the locking mechanism an application of an inward pressure to the housing (squeezing) at positions generally 90° offset to the locking elements is required. The housing is deformed outwardly in the regions of the locking elements and by simultaneously turning the carriage the locking elements disengage from the counter elements and the starting position is reached. It can be favorable to provide the inner side of the housing with four locking elements being oriented at 90° to each other. In this way, the diametrically opposed counter elements engage with the further provided locking elements after the carriage has been rotated by 90° such that a reverse rotation is prevented.

In yet another embodiment, an internal thread is provided at the housing cooperating with an external thread provided at the carriage. Such threaded sections advantageously permit a controlled rotational and axial shift of the carriage relative to the housing. In particular, on use of threaded sections the carriage can be linearly displaced relative to the housing upon a rotary motion of the carriage.

It is preferred that the external, in particular left-handed, thread of the carriage comprises two portions, wherein the portions preferably have the same pitch and are in particular of the same size and shape. It is further preferred that the portions extend across an angle of less than 180°, respectively. The complementary internal thread of the housing preferably also consists of at least two portions such that the carriage can be combined with the housing in two orientations which are essentially oriented at 180° to one another in relation to the longitudinal axis defined by the housing. It can also be favorable to provide an internal thread comprising four, particularly left-handed, thread portions oriented at 90° to each other, wherein the portions preferably have the same pitch and in particular are of the same size and shape.

Further preferably, the external and/or internal thread comprises at least one abutment flank as a rotational boundary to prevent that the carriage after having reached the locking position, i.e. the securing means being engaged, is turned further. At least one other abutment flank may be provided in order to prevent that the carriage after having reached the starting position or the discharge position, is turned further. Such abutment flanks are preferably located directly adjacent to the ramp-shaped locking elements in order to prevent a further rotation of the carriage after the locking position has been reached.

Especially in cases where the carriage has to be turned further from the starting position into the discharge position, it is preferred that an abutment flank stops the rotational movement after the discharge position has been reached. This further enhances ease of use of the discharger.

It is preferred that the starting position is reached immediately after the securing means are disengaged which approximately corresponds to an angle of rotation of the carriage between 1 ° and 90°, preferably between 5° and 70°, more preferably between 10° and 50°, measured from the locking position.

It is further preferred that the discharge position is reached after the carriage is rotated by an angle of between 10° and 200°, preferably 45° and 140°, more preferably 70° and 100°, measured from the starting position. Particularly preferred, the discharge position is reached after the carriage is rotated by an angle of approximately 90°, measured from the locking position.

Expediently, the starting position corresponds to the discharge position.

It has to be noted that due to the cooperating threads the carriage performs an axial displacement while being rotated.

In a further embodiment, the discharger comprises an intermediate piece being connected to the proximal end of a main portion of the housing and forming part of the housing. In this case the housing is formed as two-part housing comprising the main portion and the intermediate piece connected thereto. The connection can for example be established by gluing, welding, latching or any other suitable connection method.

In yet another embodiment, the intermediate piece is detachably connected to the main portion of the housing, wherein the connection can be a screw or plug-in connection, for instance.

Advantageously, the intermediate piece comprises the at least one locking element and/or the internal thread.

The two-part design of the housing enables an easy assembly of the discharger since the intermediate piece is accessible from both sides. Therefore, the intermediate piece and the carriage can be brought into contact prior to the connection of the intermediate piece to the main portion of the housing. Moreover, by providing an intermediate piece carrying portions of the locking mechanism, different versions of the discharger being child-resistant or not can be produced using identical base parts. Where appropriate, the discharger could also be applied without the intermediate piece bearing portions of the locking mechanism, i.e. securing means. In this way a high degree of modularity is achieved.

As part of the two-part housing, the intermediate piece is preferably elastically deformable at least in those regions where the first actuating force is brought about. Expediently, the intermediate piece consists of elastically deformable polymers such as polypropylene. Particularly, the intermediate piece is made of the same material as the housing. The intermediate piece, for example, can be linked with the housing by a plug connection.

In a preferred embodiment the intermediate piece and the housing are formed in one piece. In other words, it can be preferred that the housing is formed integrally with the intermediate piece.

The intermediate piece preferably is reusable since it normally does not come into contact with the fluid to be discharged.

However, more preferably, the intermediate piece is disposable, i.e. designed as disposable article intended for one-time use only. In particular, the discharger as a whole is disposable.

Furthermore, it is preferred that the carriage defines a receiving space for an amount of fluid to be discharged.

By providing a carriage with a receiving space, either a separate container containing the fluid to be discharged can be loaded into the carriage or the fluid to be discharged can be directly stored inside the receiving space. The amount of the fluid either contained in the container or in the carriage itself may for example range between 0,1 and 5 ml.

However, it is preferred that a separate container is applied to avoid a direct storage of the fluid inside the discharger and to provide for a high degree of modularity. The carriage, in this way, can be reusable, too.

In an embodiment, the fluid inside the container is protected from environmental influences by a breakable seal.

In an embodiment the discharger comprises activation means for establishing a flow connection for the fluid from within the receiving space of the carriage to the inlet opening of the discharge section.

The activation means preferably establish the flow connection for the fluid to the inlet opening of the discharge section when the carriage is transferred from the locking position into the starting position or into the discharge position. The fluid can thereby begin to flow through the discharge passage before the carriage has reached its discharge position. Alternatively, the flow connection for the fluid to the inlet opening can also be established when the carriage is transferred from the starting position into the discharge position.

Expediently, the activation means establishes a flow connection for the fluid to the inlet opening of the discharge section when the carriage is rotated from the locking position by an angle of between 10° and 200°, preferably between 45° and 140°, more preferably between 70° and 100°. Most preferably, the flow connection is established after the carriage is rotated by approximately 90°, measured from the locking position.

The activation means may also require an additional operation step to be performed by the user to establish the flow connection. The flow connection, for example, could be established only after the carriage starts its axial movement from the discharge position towards the distal end of the housing.

In an embodiment, the activation means are at least partially positioned inside the housing so as to protrude into the receiving space when the carriage is being moved from the locking position, starting position or discharge position towards the distal end of the housing. By protruding into the receiving space of the carriage, the activation means preferably interacts with a container loaded into the receiving space, in particular by breaking a seal of the container.

The activation means may be provided with means for breaking the seal. In particular, these means are adapted to provide a piercing, penetrating, puncturing and/ or perforating action for the seal.

Means such as wing-like projections can be formed at an outer side of the housing so that the discharger may be used like a common syringe by holding the discharger with one finger at each projection and with the thumb on a proximal end of the carriage or of a container loaded into the carriage, thereby providing for a comfortable single-hand operation of the discharger after the locking mechanism has been released.

Another aspect of the invention relates to a discharge system comprising a discharger as disclosed herein and at least one container holding an amount of fluid to be discharged, wherein the container is adapted to be loaded into the receiving space defined by the carriage of the discharger. An intermediate piece bearing portions of the locking mechanism as disclosed herein can be optionally contained.

A further aspect of the invention relates to the use of a discharger as disclosed herein or of a discharge system as disclosed herein, for discharging a liquid including at least one medical, dental or veterinary agent, wherein in particular the amount of the liquid lies in the range of 0,1 to 5 ml.

The present disclosure is described in the following by way of example only in connection with one embodiment with reference to the drawing comprising the following figures:
- Figs. 1 and 2: show a discharge system comprising a discharger according to the present disclosure in different perspectives,
- Figs. 3 and 4: show a discharge system as shown in Figs. 1 and 2 with securing means being engaged (Fig. 3) and securing means being disengaged (Fig. 4). For the purpose of improved visualization, the container and portions of the intermediate piece and the carriage are omitted.
- Fig. 5: shows a cross-sectional view along the section line A-A of the discharge system of Fig. 3,
- Fig. 6: shows a cross-sectional view along the section line A-A of the discharge system of Fig. 4,
- Fig. 7: shows a side view of the carriage, and
- Fig. 8: shows a perspective view of the intermediate piece.

In the following the same reference numerals will be used for parts having the same or equivalent function. Any statements made having regard to the direction of a component are made relative to the position shown in the drawing and can naturally vary in the actual position of application.

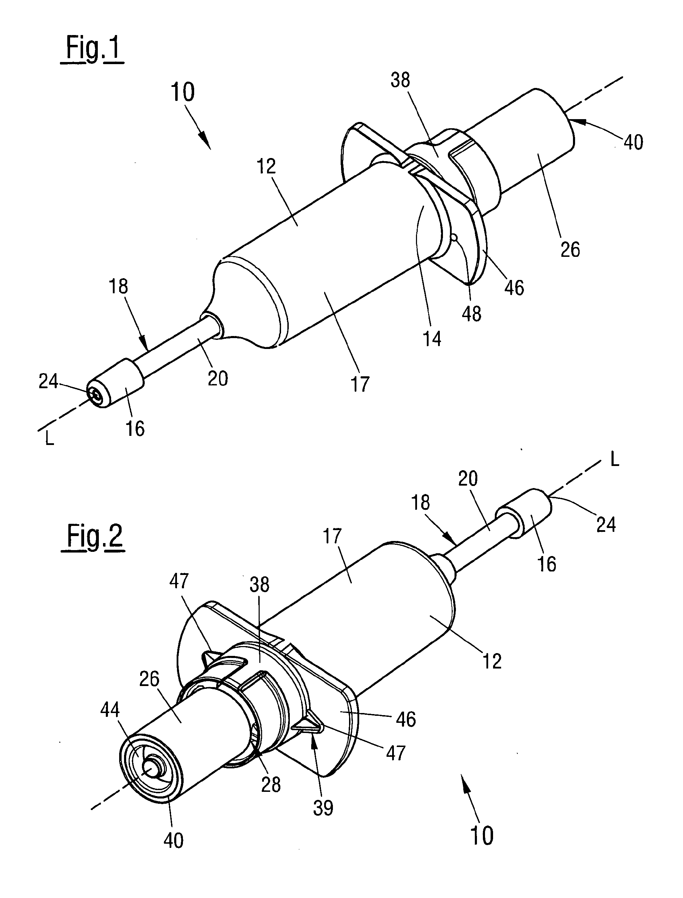

Generally, in the embodiment shown in Figs. 1 to 6, the discharge system comprises a discharger 10 having a housing 17 which is formed as a two-part housing comprising a main portion 12 and an intermediate piece 38. The intermediate piece 38 is connected to the main portion 12 of the housing 17 via connection means 39. It has to be noted in this connection that the intermediate piece 38 can also be formed integrally with the main portion 12 of the housing 17, i.e. that the housing 17 of the discharger is formed in one piece. In this case, any connection means 39 would be omitted.

Moreover, as can be seen from Figs. 2 to 6, a separate container 44 having a generally cylindrical shape can be loaded into the discharger 10 of the discharge system.

Although other appliances are possible, in the embodiment shown the discharger 10 is intended for use in the medical, dental or veterinary field, and for discharging a predetermined amount of a fluid 11 which is contained inside the container 44. The fluid 11 to be discharged, in general, is a liquid which includes one or more medical, dental or veterinary agents.

The discharger 10 and the container 44 are designed as disposable articles intended for one-time use only.

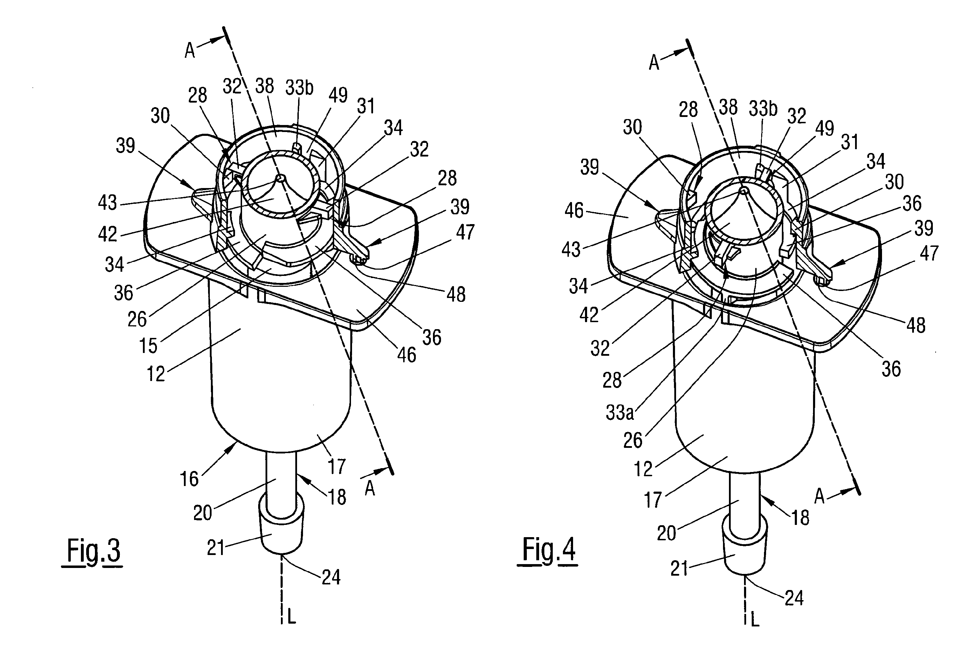

The main portion 12 of the housing 17 of the discharger 10 defines a longitudinal axis L. The discharger 10 can be designed essentially rotationally symmetrical with respect to the axis L. At its proximal end 14 the discharger 10 is provided with two wing-like protrusions 46 extending in opposite directions like in a common disposable syringe. As already mentioned above, the intermediate piece 38 is connected to the main portion 12 of the housing 17 via two connections means 39 formed as plug connections each located on one of the wing-like protrusion 46. The connections means 39 comprise two connecting pins 47 (which are shown in more detail in Figs. 3, 4 and 8) located diametrically opposed at the outer surface of the intermediate piece 38 and two corresponding holes 48 located on each of the two wing-like protrusions 46. Expediently, the main portion 12 of the housing 17 and the intermediate piece 38 have approximately the same diameter.

The discharger 10 also comprises a discharge section 18 from which Figs. 1 and 2 only show a portion of a discharge passage 20 extending from the distal end 16 of the main portion 12 of the housing 17 towards a thickened distal end portion 21. The distal end portion 21 can be formed integrally with the discharge passage 20 or made as a separate component connected in a suitable manner to the discharge passage 20. The distal end portion 21 moreover defines an outlet opening 24 through which the fluid 11 finally is discharged.

The end portion 21, in general, can be adapted to the type and site of application and can be in the form of or comprise a spray head, a needle (cannula), a brush, a sponge or a pipette. For large area applications, for example, a spray head or a sponge can be of advantage, whereas selective applications might require a needle or a plain tube of small diameters.

A carriage 26 is received in a proximal end region of the intermediate piece 38. As can be seen in Figs. 2, 5 and 6, the carriage 26 comprises a receiving space 40 into which a container 44 has been loaded.

The carriage 26 and the intermediate piece 38 comprise securing means 28 essentially constituting a locking mechanism as explained in detail below. The securing means 28 comprise locking elements 30 at the inner surface of the intermediate piece 38 and counter elements 32 at the outer surface of the carriage 26 which are shown in more detail in Figs. 3, 4, 7 and 8.

In general and notwithstanding the child-resistance, the carriage 26 is movable along the longitudinal axis L. The discharger 10 is adapted to discharge the fluid 11 inside the container 44 through the discharge section 18 and finally through the outlet opening 24 when moving the carriage 26 along the axis L towards the distal end portion 21 of the discharge section 18.

Fig. 3 shows the discharger 10 with the locking mechanism being activated and Fig. 4 shows the discharger 10 after the locking mechanism has been released. Fig. 4 corresponds to Fig. 3 after the carriage 26 has been rotated counter-clockwise by 90°. In both figures, portions of the intermediate piece 38 and the carriage 26 as well as the container 44 have been omitted for the purpose of improved visualization.

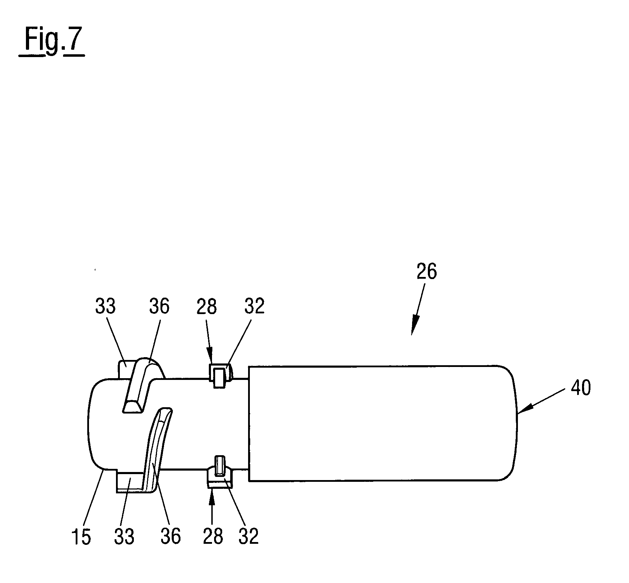

Therefore, Figs. 3 and 4 reveal that the carriage 26 on its external surface is provided with an external thread 36 which cooperates with an internal thread 34 provided at the inner surface of the intermediate piece 38. The external thread 36 comprises two left-handed thread portions oriented at 180° to each other having the same pitch and being of the same size and shape. The external thread 36 further comprises two diametrically opposed abutment flanks 33a which are located directly beneath the left-handed thread portion, respectively. This arrangement is clearly visible in Fig. 7 showing a side view of the carriage 26.

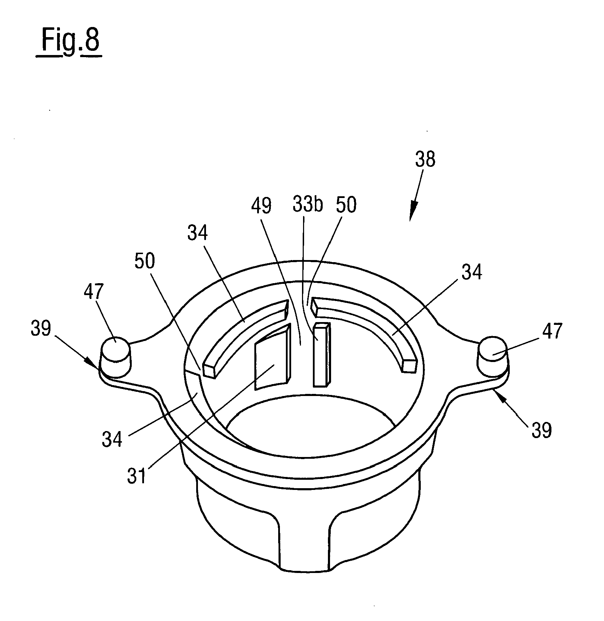

The internal thread 34 comprises four left-handed thread portions oriented at 90° to each other having the same pitch and being of the same size and shape. In other words, the internal thread 34 comprises two left-handed thread portions oriented at 180° to each other, wherein each thread portion is interrupted by a central gap or interruption. Thereby each thread portion is divided into two parts. This arrangement can also be seen in Fig. 8 showing a perspective view of the intermediate piece 38.

Also visible in Figs. 3 and 4 are activation means 42 having a flow connection 43 to the discharge passage 20. The activation means 42 are partially positioned inside a distal cylindrical portion 15 of the carriage 26.

Figs. 3 and 4 also show that the connecting pins 47 of the connection means 39 are inserted into the corresponding holes 48 on each side of the wing-like projections 46, thereby forming the plug connection between the intermediate piece 38 and the main portion 12 of the housing 17.

Moreover, from Figs. 3 and 4 it can be seen that the intermediate piece 38 comprises two diametrically opposed locking elements 30 as well as two diametrically opposed further locking elements 31 located at the inner surface of the intermediate piece 38, respectively. The further locking elements 31 are oriented at 90° to the locking elements 30, respectively. Both, the locking elements 30 and the further locking elements 31 have a ramp-like shape. However, the pitch of the ramp-shaped locking elements 30 points in the opposite direction compared to the pitch of the ramp-shaped further locking elements 31. The intermediate piece 38 further comprises at its inner surface two diametrically opposed abutment flanks 33b arranged adjacent to the ramp-shaped further locking elements 31. Both, the further locking elements 31 and the abutment flanks 33b extend axially towards the internal threads 34 until almost touching them. Thereby, the abutment flanks 33b and the further locking elements 31 form a lateral guide 49 for the counter elements 32 (shown in more detail in Fig. 8).

The counter elements 32 are located diametrically opposed at the outer surface of the carriage 26. As mentioned previously, the locking elements 30 and the counter elements 32 can collectively referred to as securing means 28. The securing means 28 and the further locking elements 31 are formed integrally with the intermediate piece 38 or the carriage 26.

When the child-resistance is activated as depicted in Fig. 3 the locking elements 30 and the counter elements 32 are engaged, i.e. the diametrically opposed counter elements 32 have been slid over the ramp-shaped locking elements 30 to arrive at the locking position. A further rotation after the locking position is reached is prevented by the abutment flanks 33a which are positioned such that they abut on one of the four left-handed thread portions of the internal thread 34 after the carriage 26 has reached the locking position (not visible in Fig. 3). Thereby, the carriage 26 is neither movable in the axial direction nor can be rotated clockwise or counter clockwise.

In order to release the child-resistance of the discharger 10 the user has to apply an inward pressure (squeezing) to the elastically deformable intermediate piece 38 at positions oriented 90° to the diametrically opposed counter elements 32. This can be achieved by holding the intermediate piece 38 between thumb and index finger and pressing them together. As a result, the intermediate piece 38 as part of the housing 17 of the discharger 10 is deformed outwardly in the regions of the locking elements 30 cooperating with the diametrically opposed counter elements 32 resulting in a disengagement of the locking elements 30 and the counter elements 32. By simultaneously rotating the carriage 26 counter clockwise through an angle of 90° the disengaged counter elements 32 can pass the locking elements 30 and the carriage 26 after passing the starting position arrives in its discharge position as depicted in Fig. 4. In the discharge position the diametrically opposed counter elements 32 are engaged with the ramped-shaped further locking elements 31 which due to their opposite pitch direction prevent a back rotation of the carriage 26. A further rotation of the carriage 26 is prevented by the abutment flanks 33b arranged adjacent to the ramp-shaped further locking elements 31.

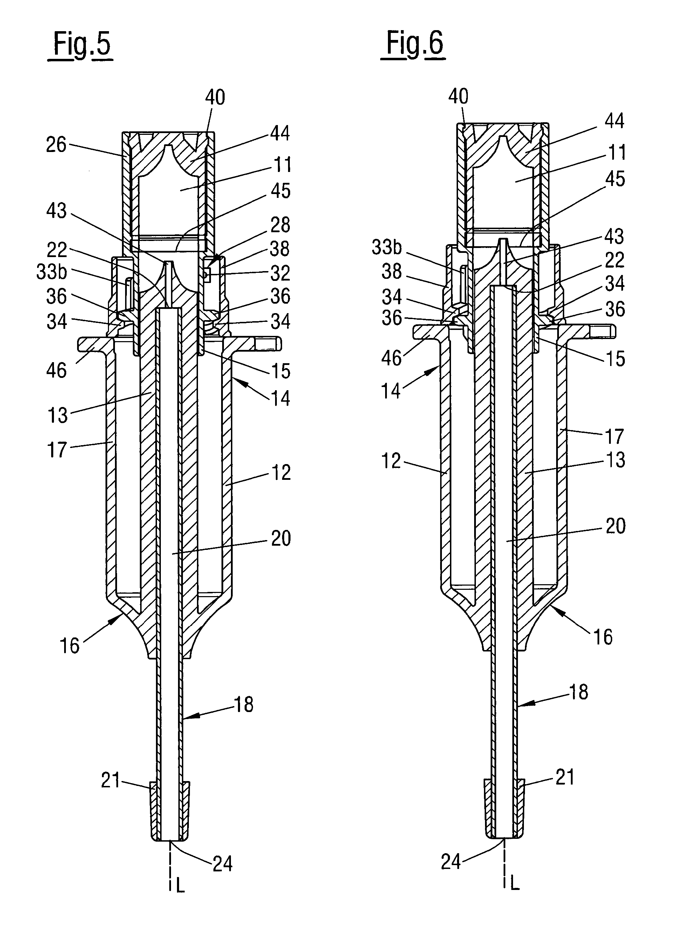

Fig. 8 clearly shows one of the further locking elements 31 and one of the abutment flanks 33b located at the inner surface of the intermediate piece 38 which has been turned upside down compared to its orientation in Figs 4 and 5. The abutment flanks 33b and the further locking elements 31 are designed and arranged such that they form the lateral guide 49 being aligned with an interruption 50 between the thread portions of the internal threads 34. The lateral guide 49 and the interruption 50 ensure that the carriage 26 after having reached the discharge position can be moved axially towards the distal end 16 by providing a path for the counter elements 32. Also clearly visible in Fig 8 are the connecting pins 47 which extend in axial direction from the intermediate piece 38.

Along with the rotation by 90°, the carriage 26 additionally performs an axial movement towards the distal end 16 of the discharger 10 due to the threaded connection between the carriage 26 and the intermediated piece 38. As a result, the container 44 (not shown in Fig. 3 and 4) moves towards the activation means 42 which pierces a seal 45 closing the distal end of the container 44. In order to discharge the fluid 11 when having reached the discharge position, the user must deliberately push the carriage 26 towards the distal end 16.

The axial movement of the carriage 26 when transferred from the locking position into the discharge position becomes more obvious from Fig. 5 showing a cross-sectional view (along section line A-A) of the discharger 10 of Fig. 3 in its locking position and from Fig. 6 showing a cross-sectional view (along section line A-A) of the discharger 10 of Fig. 4 in its discharge position. Fig. 6, accordingly, corresponds to Fig. 5 after the carriage has been rotated by 90° counter-clockwise.

In Fig. 5 the locking elements 30 and the counter elements 32 are engaged, i.e. the carriage 26 is in the locking position. The receiving space 40 of the carriage 26 contains the container 44 which is filled with the fluid 11 to be discharged. The container 44 is made as a capsule which has a cylindrical shape. Its opening is closed by a breakable seal 45 in the form of a foil. The amount of the fluid 11 inside the container 44 is approximately 0,5 ml.

The main portion 12 of the housing 17 comprises a sleeve portion 13 formed integrally with the main portion 12. Further, the main portion 12 of the housing 17 comprises the discharge passage 20 which is fitted into the sleeve portion 13. A proximal end portion of the sleeve portion 13 is fitted into the distal cylindrical portion 15 of the carriage 26 through a distal opening thereof. The sleeve portion 13 comprises an inlet opening 22 at its proximal end portion and the activation means 42. The activation means 42 comprise a tip and a flow connection 43 establishing a connection to the discharge passage 20. The tip is formed by a correspondingly tapering of the proximal end portion of the sleeve portion 13.

In the locking position, the activation means 42 is positioned spaced apart from the seal 45 of the container 44, i.e. the activation means 42 comprising the tip do not yet protrude into the receiving space 40 of the carriage 26 being loaded with the container 44.

For reaching the discharge position as depicted in Fig. 6, the user at first has to unlock the child-resistance by squeezing the intermediate piece 38 at diametrically opposed positions and simultaneously rotating the carriage 26 by 90° as has been described with respect to Figs. 3 and 4. Due to the cooperating internal and external threads 34, 36 the carriage 26 moves towards the distal end 16 and the activation means 42 thereby at least partially break the seal 45 and establish a flow connection 43 for the fluid 11 inside the container 44 to the inlet opening 22.

For discharging the fluid 11, the user has to move the carriage 26 along the longitudinal axis L into the main portion 12 of the housing 17 by pushing it deliberately. While the carriage 26 continues its movement, the activation means 42 enters into the fluid space of the container 44, thereby urging the fluid 11 out of the container 44. The outer shape of the tapered proximal end portion of the sleeve portion 13, i.e. the activation means 42, essentially corresponds to the inner dimensions of the container 44 so that almost no free space remains inside the container 44 when the activation means 42 moves into the fluid space of the container 44. The fluid 11 can only escape through the flow connection 43 and inlet opening 22 and thus through the discharge passage 20 of the discharger 10.The complementary shapes of the activation means 42 and the container 44 ensure that essentially no residual amounts of fluid 11 remain within the container 44.

Generally, the discharger 10 and/or the container 44 may be fabricated from any suitable material. In one embodiment, the material is plastic. The material may be selected from the group comprising polypropylene, cyclic olefin polymer, polyethylene, polyamide, polybutylenterephthalat and polymethyl methacrylate. Alternatively, the material may be glass, metal or an alloy.

It is particularly preferred that the discharger 10 and/or the container 44 are fabricated by injection molding as injection-molded parts.

With respect to one common classification applied in the medical, dental or veterinary field or the health care sector, the discharger and the discharge system as provided by the present disclosure belong to the group consisting of systems without protection cap.

### List of reference numerals:

- 10: discharger
- 11: fluid
- 12: main portion of the housing
- 13: sleeve portion
- 14: proximal end (region)
- 15: distal cylindrical portion of the carriage
- 16: distal end
- 17: housing
- 18: discharge section
- 20: discharge passage
- 21: distal end portion
- 22: inlet opening
- 24: outlet opening
- 26: carriage
- 28: securing means
- 30: locking element
- 31: further locking element
- 32: counter element
- 33a, 33b: abutment flanks
- 34: internal thread
- 36: external thread
- 38: intermediate piece
- 39: connection means
- 40: receiving space
- 42: activation means
- 43: flow connection
- 44: container
- 45: seal
- 46: wing-like projections
- 47: connecting pin
- 48: hole
- 49: lateral guide
- 50: interruption

- L: longitudinal axis

## Claims

1. Discharger (10) for discharging a predetermined amount of fluid (11) comprising
- a housing (17) defining a longitudinal axis (L) and having a proximal end (14) and a distal end (16),
- a discharge section (18) having a discharge passage (20) for the fluid (11) extending between an inlet opening (22) and an outlet opening (24) of the discharge section (18),
- a carriage (26) cooperating with the proximal end region (14) of the housing (17) such that the carriage (26) is moveable relative to the housing (17) along the longitudinal axis (L), and
- securing means (28) being engageable with each other such that they define a locking position in which the carriage (26) is prevented from being moved along the longitudinal axis (L), the securing means (28) being disengageable to define a starting position for the carriage (26) by simultaneously applying a first actuating force to the housing (17) and a second actuating force to the carriage (26).

2. Discharger according to claim 1, wherein the first actuating force is brought about by a radial compression of the housing (17), preferably at its proximal region (14).

3. Discharger according to claim 1 or 2, wherein the second actuating force is brought about by a rotary motion or an axial motion of the carriage (26) relative to the housing (17) or a combination of both.

4. Discharger according to any of the preceding claims, wherein the securing means (28) comprise at least one locking element (30) provided on the housing (17) and at least one counter element (31) provided on the carriage (26).

5. Discharger according to claim 4, wherein the locking element (30) is adapted to slide over the counter element (32) when the carriage (26) is rotated relative to the housing (17) in a first direction for arriving at the locking position, and wherein the locking element (30) and the counter element (32) prevent a rotation in the direction opposite to the first direction when they are engaged in the locking position.

6. Discharger according to claim 5, wherein the housing (17) is deformable in order to disengage the locking element (30) and the counter element (32) by radially compressing the housing (17) and rotating the carriage (26) in the direction opposite to the first direction.

7. Discharger according to any of claims 4 to 6, wherein the discharger (10) comprises an intermediate piece (38) being connected to the proximal end (14) of a main portion (12) of the housing (17) and forming part of the housing (17), wherein the intermediate piece (38) comprises the at least one locking element (30).

8. Discharger according to any of the preceding claims, wherein an internal thread (34) is provided at the housing (17) cooperating with an external thread (36) provided at the carriage (26).

9. Discharger according to claim 8, wherein the discharger (10) comprises an intermediate piece (38) being connected to the proximal end (14) of the housing (17) and forming part of the housing (17), wherein the intermediate piece (38) comprises the internal thread (34).

10. Discharger according to any of the preceding claims, wherein the carriage (26) defines a receiving space (40) for an amount of fluid (11) to be discharged.

11. Discharger according to claim 10, wherein the discharger (10) comprises activation means (42) for establishing a flow connection for the fluid (11) from within the receiving space (40) of the carriage (26) to the inlet opening (22) of the discharge section (18).

12. Discharger according to claim 11, wherein the activation means (42) establish the flow connection (43) for the fluid (11) to the inlet opening (22) of the discharge section (18) when the carriage (26) is transferred from the locking position into the starting position.

13. Discharger according to claim 11, wherein the activation means (42) establish the flow connection for the fluid (11) to the inlet opening (22) of the discharge section (18) when the carriage (26) is rotated from the locking position to the starting position.

14. Discharge system comprising
- a discharger (10) according to any of claims 1 to 13, and
- at least one container (44) holding an amount of fluid (11) to be discharged, the fluid preferably containing at least one medical, dental and/or veterinary agent, wherein the container (44) is adapted to be loaded into the receiving space (40) defined by the carriage (26) of the discharger (10).

15. Use of a discharger 10 according to any of claims 1 to 13 or of a discharge system according to claim 14, for discharging a fluid (11) including at least one medical, dental or veterinary agent, wherein in particular the amount of the fluid (11) lies in the range of 0,1 to 5 ml.
